# EUROPEAN PATENT APPLICATION

(11) **EP 4 541 281 A1**
(43) Date of publication of application: **23.04.2025**
(21) Application number: 23843430.2
(22) Date of filing: 21.07.2023
(51) Int. Cl.: A61B 5/346, A61B 5/00, G16H 50/20

(54) **METHOD, PROGRAM, AND DEVICE FOR DIAGNOSING MYOCARDIAL INFARCTION USING ELECTROCARDIOGRAM**

(30) Priority: 22.07.2022 KR 20220090768; 18.07.2023 KR 20230093192
(71) Applicant: Medical AI Co., Ltd., Seoul 06180 (KR)
(72) Inventor: KWON, Joonmyoung, Seoul 06180 (KR); LEE, Minsung, Seoul 06180 (KR); SON, Jeongmin, Seoul 06180 (KR)
(74) Representative: Patentanwaltskanzlei Matschnig & Forsthuber OG
(86) International application number: PCT/KR2023/010577
(87) International publication number: WO 2024/019584

(57) **Abstract**

According to an embodiment of the present disclosure, a method, program, and device for diagnosing myocardial infarction using an electrocardiogram, performed by a computing device, are disclosed. The method may include generating training data based on electrocardiogram data of a patient who undergoes coronary angiography; and training a deep learning model that predicts an onset or progression of myocardial infarction in the patient using the generated training data.

## Description

### [Technical Field]

The present disclosure relates to an artificial intelligence technology in the field of medicine, and more particularly, to a method of training a model for diagnosing myocardial infarction using an electrocardiogram and using the trained model.

### [Background Art]

An electrocardiogram is a test that records electrical activity of a heart. An electrocardiogram is a relatively simple and cost-effective test method of checking a health status of a heart, and plays an important role in the early diagnosis and management of heart disease. For example, an electrocardiogram signal measured through an electrocardiogram test may be used to check whether each part of the heart is functioning normally, whether a size and location of the heart are normal, and whether there is damage to a heart muscle, etc. In addition, an electrocardiogram signal may be used to diagnose various heart-related problems and predict a person's health status based on the check.

Meanwhile, as an artificial intelligence (AI) technology develops, attempts to analyze an electrocardiogram using the artificial intelligence technology are increasing. In particular, there are many studies on models that diagnose arrhythmia using an electrocardiogram. However, unlike arrhythmia, there are almost no studies on models that diagnose myocardial infarction. Moreover, myocardial infarction with 80% to 90% blockage of the coronary artery is known to be difficult to detect even for medical professionals using only an electrocardiogram. Therefore, it is necessary to apply an artificial intelligence technology in the field of diagnosing myocardial infarction using an electrocardiogram.

### [Disclosure]

### [Technical Problem]

The present disclosure provides a method of diagnosing myocardial infarction by training a model that diagnoses myocardial infarction using an electrocardiogram and using the trained model.

However, the problems to be solved by the present disclosure are not limited to the problems described above, and other problems that are not mentioned may be clearly understood based on the description below.

### [Technical Solution]

According to an embodiment of the present disclosure, a method of diagnosing myocardial infarction using an electrocardiogram, performed by a computing device is disclosed. The method may include generating training data based on electrocardiogram data of a patient who undergoes the coronary angiography; and training a deep learning model that predicts an onset or progression of myocardial infarction in the patient using the generated training data.

The generating of the training data based on the electrocardiogram data of the patient who undergoes the coronary angiography may include labeling a degree of stenosis of a coronary artery in the electrocardiogram data of the patient who undergoes the coronary angiography to generate the training data.

The labeling may be performed differently according to the type of coronary artery.

The training of the deep learning model that predicts the onset or progression of myocardial infarction in the patient using the generated training data may include: classifying the training data based on at least one of a first criterion indicating demographics, a second criterion indicating a factor affecting the onset or progression of myocardial infarction, a third criterion indicating a type of disease and subject of onset, or a fourth criterion indicating a type of electrocardiogram to generate subgroups; and training the deep learning model to predict the onset or progression of myocardial infarction for each of the generated subgroups.

The training of the deep learning model that predicts the onset or progression of myocardial infarction in the patient using the generated training data may include: selecting a group whose performance is less than or equal to a reference value based on a validation result derived from the training process of the generated subgroups; and performing additional training on the deep learning model on which the training is performed based on the selected group.

The additional training may be performed based on few shot learning.

The second group may include at least one of a risk factor for disease onset, a medical history, whether one is an early onset patients or not, or a type of chest pain.

The third group may include at least one of a type of myocardial infarction, a type of acute coronary syndrome, or a type of coronary artery.

According to another embodiment of the present disclosure, a method of diagnosing myocardial infarction using an electrocardiogram, performed by a computing device is disclosed. The method may include: acquiring electrocardiogram data; and inputting the electrocardiogram data to a pre-trained deep learning model to predict an onset or progression of myocardial infarction of a subject whose electrocardiogram data is measured. The deep learning model may be pre-trained to predict the onset or progression of myocardial infarction of a patient using training data generated based on the electrocardiogram data of the patient who undergoes coronary angiography.

According to still another embodiment of the present disclosure, a computer program stored in a computer-readable storage medium is disclosed. The computer program performs operations for diagnosing a myocardial infarction using an electrocardiogram when executed on one or more processors. The operations may include: generating training data based on electrocardiogram data of a patient who undergoes the coronary angiography; and training a deep learning model that predicts an onset or progression of myocardial infarction in the patient using the generated training data.

According to yet another embodiment of the present disclosure, a computing device for diagnosing myocardial infarction using an electrocardiogram is disclosed. The device may include: a processor including at least one core; a memory including program codes executable in the processor; and a network unit acquiring electrocardiogram data. The processor may generate training data based on electrocardiogram data of a patient who undergoes coronary angiography, and train the deep learning model that predicts the onset or progression of myocardial infarction in the patient using the generated training data.

### [Advantageous Effects]

According to the method of the present disclosure, it is possible to analyze not only the presence or absence of the myocardial infarction disease but also how the myocardial infarction is progressing through an artificial intelligence model.

In addition, it is possible to supplement the shortcomings of a model that shows insufficient performance in a specific patient group through few shot learning for the artificial intelligence model described above.

### [Description of Drawings]

FIG. 1 is a block diagram of a computing device according to an embodiment of the present disclosure.
FIG. 2 is a block diagram illustrating a process of training a deep learning model according to an embodiment of the present disclosure.
FIG. 3 is a block diagram illustrating a process of training a deep learning model according to another embodiment of the present disclosure.
FIG. 4 is a flowchart illustrating a method of training a deep learning model according to an embodiment of the present disclosure.
FIG. 5 is a flowchart illustrating an inference method of a deep learning model according to an embodiment of the present disclosure.

### [Best Mode]

Hereinafter, embodiments of the present disclosure will be described in detail with reference to the attached drawings so that those skilled in the art can easily implement the present disclosure. The embodiments presented in the present disclosure are provided so that those skilled in the art may utilize or implement contents of the present disclosure. Accordingly, various modifications to the embodiments of the present disclosure will be apparent to those skilled in the art. That is, the present disclosure may be implemented in various different forms, and is not limited to embodiments described below.

Throughout the specification of the present disclosure, the same or similar drawing symbols refer to the same or similar components. In addition, in order to clearly describe the present disclosure, drawing symbols of parts that are not related to the description of the present disclosure may be omitted in the drawings.

The term "or" used in the present disclosure is intended to mean an inclusive "or," not an exclusive "or." That is, unless otherwise specified in the present disclosure or the meaning is clear from the context, "X uses A or B" is intended to mean one of the natural inclusive substitutions. For example, unless otherwise specified in the present disclosure or the meaning is clear from the context, "X utilizes A or B" may be construed as one of X utilizing A, X utilizing B, or X utilizing both A and B."

The term "and/or" used in the present disclosure should be understood to refer to and include all possible combinations of one or more of the related concepts listed.

The terms "include" and/or "including" used in the present disclosure should be understood to mean that specific features and/or components are present. However, the terms "include" and/or "including" should be understood as not excluding the presence or addition of one or more other features, components and/or groups thereof.

In addition, unless otherwise specified in the present disclosure or the context is clear to indicate a singular form, the singular form should generally be construed to include "one or more."

The term "N^{th} (N is a natural number)" used in the present disclosure may be understood as an expression used to mutually distinguish components of the present disclosure according to a predetermined standard such as a functional perspective, a structural perspective, or convenience of description. For example, components performing different functional roles in the present disclosure may be distinguished as a first component or a second component. However, components that are substantially the same within the technical idea of the present disclosure but should be distinguished for convenience of description may also be distinguished as a first component or a second component.

The term "acquisition" used in the present disclosure may be understood to mean not only receiving data through a wired or wireless communication network with an external device or system, but also generating data in an on-device form.

Meanwhile, the term "module" or "unit" used in the present disclosure may be understood as a term referring to an independent functional unit that processes computing resources, such as a computer-related entity, firmware, software or a part thereof, hardware or a part thereof, or a combination of software and hardware. In this case, the "module" or "unit" may be a unit composed of a single element or may be a unit expressed as a combination or set of multiple elements. For example, as a narrow concept, "module" or "unit" may refer to hardware elements of a computing device or a set thereof, an application program that performs a specific function of software, a processing process implemented through software execution, a set of instructions for program execution, etc. In addition, as a broad concept, "module" or "unit" may refer to a computing device itself that constitutes a system, an application that is executed on the computing device, etc. However, the above-described concept is only an example, and the concept of "module" or "unit" may be variously defined within a category understandable to those skilled in the art based on the content of the present disclosure.

The term "model" used in the present disclosure may be understood as a system implemented using mathematical concepts and language to solve a specific problem, a set of software units to solve a specific problem, or an abstraction model of a processing process to solve a specific problem. For example, a neural network "model" may be the entire system implemented as a neural network that has a problem-solving ability through learning. In this case, the neural network may obtain its problem-solving ability by optimizing parameters connecting nodes or neurons through learning. The neural network "model" may include a single neural network or may include a neural network set that combines a plurality of neural networks.

The description of the above-described terms is intended to help understanding of the present disclosure. Therefore, when the above-described terms are not explicitly described as matters limiting the contents of the present disclosure, it should be noted that they are not used in a sense limiting the technical ideas of the contents of the present disclosure.

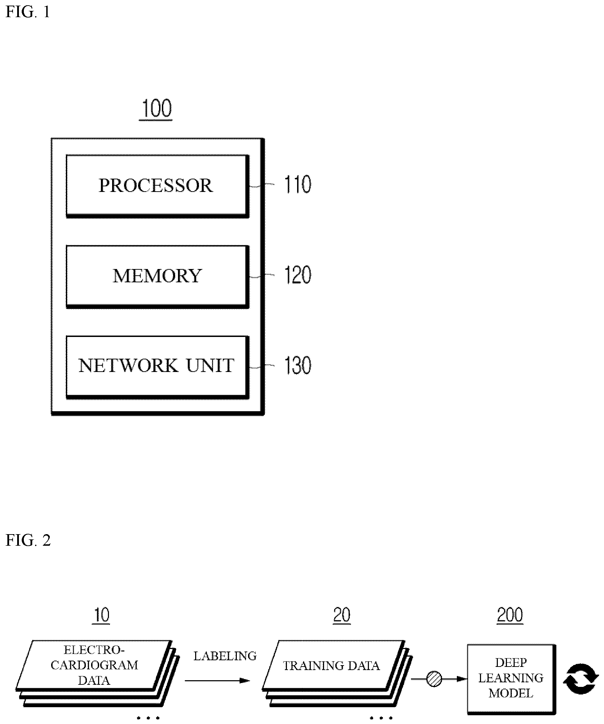

FIG. 1 is a block configuration diagram of a computing device according to an embodiment of the present disclosure.

A computing device 100 according to an embodiment of the present disclosure may be a hardware device or a part of a hardware device that performs comprehensive processing and calculation of data, or may be a software-based computing environment connected to a communication network. For example, the computing device 100 may be a server that performs intensive data processing functions and shares resources, or may be a client that shares resources through interaction with a server. In addition, the computing device 100 may be a cloud system in which a plurality of servers and clients interact to comprehensively process data. Since the above description is only one example related to the type of the computing device 100, the type of the computing device 100 may be configured in various ways within a category understandable to those skilled in the art based on the contents of the present disclosure.

Referring to FIG. 1, the computing device 100 according to an embodiment of the present disclosure may include a processor 110, a memory 120, and a network unit 130. However, FIG. 1 is only one example, and the computing device 100 may include other configurations for implementing a computing environment. In addition, only some of the configurations disclosed above may be included in the computing device 100.

The processor 110 according to an embodiment of the present disclosure may be understood as a configuration unit including hardware and/or software for performing computing operations. For example, the processor 110 may read a computer program and perform data processing for machine learning. The processor 110 may process computational processes such as processing input data for machine learning, feature extraction for machine learning, and error calculation based on backpropagation. The processor 110 for performing such data processing may include a central processing unit (CPU), a general purpose graphics processing unit (GPGPU), a tensor processing unit (TPU), an application specific integrated circuit (ASIC), or a field programmable gate array (FPGA), etc. The type of the processor 110 described above is only one example, and thus the type of the processor 110 may be configured in various ways within a category understandable to those skilled in the art based on the contents of the present disclosure.

The processor 110 may generate training data for a deep learning model for diagnosing myocardial infarction based on electrocardiogram data of a patient who has undergone coronary angiography. The processor 110 may generate training data by labeling a degree of stenosis of the coronary artery on the electrocardiogram data of a patient who has undergone coronary angiography. In this case, the degree of stenosis of the coronary artery is information confirmed by performing the coronary angiography, and may be acquired together when acquiring the electrocardiogram data. That is, the computing device 100 may label the degree of stenosis of the coronary artery of the patient as the input of the electrocardiogram data of the patient who has undergone the coronary angiography to generate the training data.

The processor 110 may train a deep learning model for diagnosing myocardial infarction using the generated training data. The processor 110 may train the deep learning model to predict the onset or progression of myocardial infarction of the patient based on the training data. For example, the processor 110 may input the electrocardiogram data included in the training data to the deep learning model to output the degree of stenosis of the coronary artery for predicting the onset or progression of myocardial infarction of the patient. In this case, the output may be in the form of a score as a numerical value for expressing the degree of stenosis of the coronary artery. The processor 110 may compare the output of the deep learning model with the label to adjust parameters of the neural network included in the deep learning model. The processor 110 may train the deep learning model by repeatedly performing the above-described process until the error between the output of the deep learning model and the label satisfies a minimum criterion.

The processor 110 may predict the onset or progression of myocardial infarction using the trained deep learning model. The processor 110 may input the electrocardiogram data to the trained deep learning model to output the degree of stenosis of the coronary artery for predicting the onset or progression of myocardial infarction. In this case, the electrocardiogram data input to the deep learning model is not limited to data of the patient who has undergone the coronary angiography, and may include all the electrocardiogram data measured for various purposes in a medical environment or real life. The processor 110 may predict whether the myocardial infarction has occurred or to what degree the myocardial infarction has progressed if the myocardial infarction has occurred based on the degree of stenosis of the coronary artery output by the deep learning model.

The memory 120 according to the embodiment of the present disclosure may be understood as a configuration unit including hardware and/or software for storing and managing data processed in the computing device 100. That is, the memory 120 may store any type of data generated or determined by the processor 110 and any type of data received by the network unit 130. For example, the memory 120 may include at least one of storage media such as a flash memory type, a hard disk type, a multimedia card micro type, a card type memory (for example, an SD or XD memory, or the like), a random access memory (RAM), a static random access memory (SRAM), a read-only memory (ROM), an electrically erasable programmable read-only memory (EEPROM), a programmable read-only memory (PROM), a magnetic memory, a magnetic disk, and an optical disc. In addition, the memory 120 may include a database system that controls and manages data in a predetermined system. Since the type of the memory 120 described above is only one example, the type of the memory 120 may be configured in various ways within a category understandable to those skilled in the art based on the content of the present disclosure.

The memory 120 may structure, organize, and manage data necessary for performing an operation, combinations of the data, program codes executable by the processor 110, etc. For example, the memory 120 may store medical data received through the network unit 130 to be described below. The memory 120 may store a program code that operates a neural network model to receive medical data and perform training, a program code that operates the neural network model to receive the medical data and perform inference according to the purpose of use of the computing device 100, and processed data generated as the program codes are executed, etc.

The network unit 130 according to the embodiment of the present disclosure may be understood as a configuration unit that transmits and receives data through any known wired/wireless communication system. For example, the network unit 130 may transmit and receive data using wired or wireless communication systems such as a local area network (LAN), Wideband Code Division Multiple Access (WCDMA), Long Term Evolution (LTE), wireless broadband Internet (WiBro), 5th generation mobile communication (5G), ultra wide-band, ZigBee, radio frequency (RF) communication, a wireless LAN, wireless fidelity (WIFI), near field communication (NFC), or Bluetooth. Since the above-described communication systems are only examples, the wired and wireless communication system for data transmission and reception of the network unit 130 may be applied in various ways other than the above-described examples.

The network unit 130 may receive data necessary for the processor 110 to perform operations through the wired or wireless communication with any system or any client, etc. In addition, the network unit 130 may transmit data generated by the operation of the processor 110 through the wired or wireless communication with any system, any client, etc. For example, the network unit 130 may receive medical data through communication with a cloud server, clients such as a smart watch, or medical computing devices, etc., that perform tasks such as database within a clinical environment and standardization of medical data. The network unit 130 may transmit output data of the neural network model and intermediate data, processed data, etc., derived from the operation process of the processor 110 through the communication with the above-described database, server, client, computing device, etc.

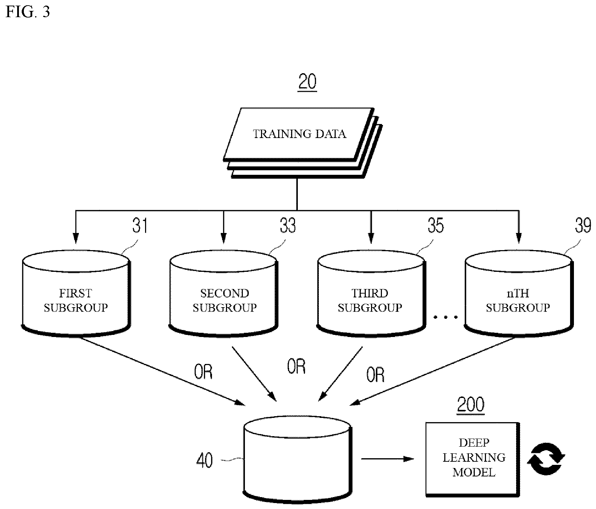

FIG. 2 is a block diagram illustrating a process of training a deep learning model according to an embodiment of the present disclosure. FIG. 3 is a block diagram illustrating a process of training a deep learning model according to another embodiment of the present disclosure.

Referring to FIG. 2, a computing device 100 according to an embodiment of the present disclosure may label a degree of stenosis of the coronary artery of a patient who has undergone coronary angiography on electrocardiogram data 10 to generate the training data 20. In this case, the computing device 100 may label the degree of stenosis of the coronary artery by distinguishing the type of coronary artery. For example, the computing device 100 may label the degree of stenosis for each classification on the electrocardiogram data 10 based on medical classifications of coronary arteries, such as right coronary artery (RCA), left anterior descending artery (LAD), and left circumflex artery (LCx). Through such labeling, it is possible to analyze which blood vessel is narrowed to what degree during the process of training the deep learning model.

The computing device 100 may input the training data 20 to the deep learning model 200 and train the deep learning model 200 to output the degree of coronary stenosis for diagnosing myocardial infarction. In this case, the deep learning model 200 may include a neural network that extracts features related to electrocardiogram waveforms, such as P waves, QRS complexes, and T waves, from an electrocardiogram and a neural network that outputs a numerical value expressing the degree of coronary stenosis based on the extracted features. For example, the computing device 100 may input the electrocardiogram data included in the training data 20 to the deep learning model 200 and output the numerical value expressing the degree of coronary stenosis. The computing device 100 may calculate an error using a loss function that uses the numerical value output by the deep learning model 200 and the label included in the training data 20 as input variables. The computing device 100 may adjust parameters of the neural network included in the deep learning model 200 based on the calculated error. **In** addition, the computing device 100 may repeatedly perform the process of adjusting the parameters of the neural network until the error satisfies the minimum criterion. The training of the deep learning model 200 may be performed based on supervised learning as in the example described above, but may also be performed based on self-supervised learning depending on the neural network structure of the deep learning model 200.

Meanwhile, when the deep learning model 200 has not been sufficiently trained on an electrocardiogram of a patient with myocardial infarction, the accuracy of the output may be low because the electrocardiogram data of the patient with myocardial infarction is not sufficiently trained. In general, the amount of electrocardiogram data of patients with diseases is smaller than that of electrocardiogram data of people in a normal healthy state. Therefore, there is a possibility that the amount of data for training the deep learning model 200 for diagnosing diseases such as myocardial infarction may not be sufficiently secured. Therefore, in order to improve the performance of the deep learning model 200, the computing device 100 may perform additional training on a data group with low output accuracy based on a verification evaluation for a specific data group confirmed during the training process.

Referring to FIG. 3, the computing device 100 may classify the training data 20 into several subgroups based on medically used factors for diagnosing the myocardial infarction. For example, the computing device 100 may classify the training data 20 into a first subgroup 31, a second subgroup 33, a third subgroup 35, and an Nth subgroup 39, which match each of various criteria affecting the diagnosis of the myocardial infarction. Specifically, the classification criteria matching each of the subgroups 31, 33, 35, and 39 may include a first criterion representing demographics, a second criterion representing factors affecting the onset or progression of myocardial infarction, a third criterion representing a type of diseases and subjects of onset, and a fourth criterion representing a type of electrocardiograms. The second criterion may include at least one of a risk factor for disease onset, a medical history, whether one is an early onset patient or not, or a type of chest pain. In addition, the third criterion may include at least one of a type of myocardial infarction, a type of acute coronary syndrome, or a type of coronary artery. The computing device 100 may classify the data included in the training data 20 according to any one of the first to fourth criteria to generate the subgroups 31, 33, 35, and 39.

**[Table 1]**

| Criterion | Large category | Medium category |
|---|---|---|
| First criterion | Demographics | Age (>65 year-old or <=65 year-old) |
| | | Sex (male or female) |
| | | BMI |
| Second criterion | Risk factor for disease onset | Hypertension |
| | | Diabetes |
| | | Hyperlipidemia |
| | | Smoking |
| | Medical history | CAD |
| | | AMI |
| | | PCI |
| | | CABG |
| | | CHF |
| | | CKD |
| | | Stroke |
| | | PAD |
| | Early onset patient or not | <3hr |
| | | >=3hr |
| | Type of chest pain | Typical chest pain |
| | | atypical chest pain |
| | | Non-cardiac chest pain |
| Third criterion | Type of myocardial infarction | Type 1 MI |
| | | Type 2 MI |
| | | Type 3 MI |
| | | Type 4 MI |
| | | Type 5 MI |
| | Type of acute coronary syndrome | STEMI |
| | | NSTEMI |
| | | UA: Unstable angina |
| | | Acute coronary syndrome (STEMI+NSTEMI+UA ) |
| | Type of coronary artery | LM |
| | | LAD |
| | | LCX |
| | | RCA |
| Fourth criterion | ECG type | LBBB |
| | | RBBB |
| | | LVH |
| | | Pacemaker rhythm |
| | | Atrial fibrillation |
| | | Atrial flutter |

The specific details of each criterion may be organized as in Table 1. However, since Table 1 is only an example, the criteria of the present disclosure are not limited to Table 1.

The computing device 100 may perform first training to allow the deep learning model 200 to predict the onset or progression of myocardial infarction for each subgroup. The computing device 100 may perform the first training to input each subgroup to the deep learning model 200 and output the degree of stenosis of the coronary artery for predicting the onset or progression of myocardial infarction. In this case, the first training may correspond to the training process of FIG. 2 described above.

The computing device 100 may derive a verification result for each subgroup during the process of performing the first training. **In** addition, the computing device 100 may select a group with low performance among the subgroups based on the verification result. For example, the computing device 100 may select a group whose performance is less than or equal to a reference value among the subgroups based on the result value of the verification performed during the process of performing the first training. The computing device 100 may select a group whose performance is less than or equal to a reference value among the subgroups based on a performance indicator used in a hospital or emergency room.

The computing device 100 may perform second training on the deep learning model 200 for which the first training has been completed based on the selected group. The computing device 100 may input a group whose performance is less than or equal to a reference value to the deep learning model 200 for which the first training has been completed, and perform additional training for improving the performance of the group based on the few-shot learning. Here, the few-shot learning is a learning method of performing inference based on a small amount of data, and may be understood as a method of training on classes of support data to which query data belongs by training on similarities between small amounts of data. That is, since the group whose performance is less than or equal to a reference value is a small amount of data, the computing device 100 may perform the second training based on the few-shot learning to input a group whose performance is less than or equal to the reference value, which is a small amount of data, to the deep learning model 200 that has completed the first training to predict the degree of stenosis of the coronary artery. Through this second training, the computing device 100 may supplement the performance of the deep learning model 200 that shows insufficient performance in a specific condition or diagnostic element.

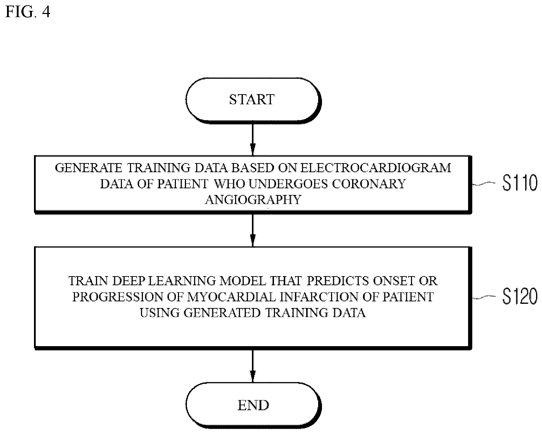

FIG. 4 is a flowchart illustrating a method of training a deep learning model according to an embodiment of the present disclosure.

Referring to FIG. 4, the computing device 100 according to an embodiment of the present disclosure may generate the training data based on electrocardiogram data of a patient who has undergone coronary angiography (S110). When the computing device 100 is a server that performs electrocardiogram reading, the computing device 100 may acquire electrocardiogram data of a patient who has undergone coronary angiography by communicating with a database within a medical environment that manages the electrocardiogram data of the patient. When the computing device 100 is a server that manages electrocardiogram data within a medical environment, if the measurement of the electrocardiogram signal of the patient who has undergone the coronary angiography is completed, the computing device 100 may generate and manage the electrocardiogram data of the patient. The computing device 100 may label the degree of stenosis of the coronary artery of the patient on the electrocardiogram data of the patient who has undergone the coronary angiography to generate the training data. In addition, when labeling the degree of stenosis of the coronary artery, the computing device 100 may perform labeling by distinguishing the type of coronary artery.

The computing device 100 may train the deep learning model that predicts the onset or progression of myocardial infarction of the patient using the training data generated through operation S110 (S120). The computing device 100 may train the deep learning model through the first training based on supervised learning, self-supervised learning, etc., according to the structure of the deep learning model. The computing device 100 may determine a data set with low prediction accuracy of the deep learning model and perform additional training on the deep learning model through the second training based on the few-shot learning. For this training, the computing device 100 may use the training data by classifying the training data in various ways based on factors related to the diagnosis of the myocardial infarction. Specifically, the computing device 100 may input each of the subgroups generated through the classification of the training data to the deep learning model to perform the first training. The computing device 100 may select a group with low training performance based on the verification result for each subgroup confirmed through the first training. The computing device 100 may perform the second training on the deep learning model based on the selected group.

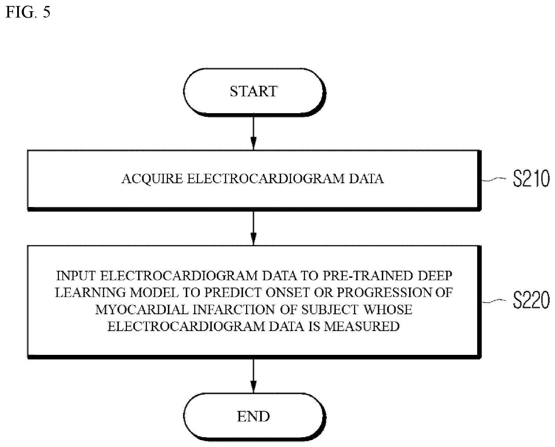

FIG. 5 is a flowchart illustrating an inference method of a deep learning model according to an embodiment of the present disclosure.

Referring to FIG. 5, the computing device 100 according to an embodiment of the present disclosure may acquire the electrocardiogram data (S210). When the computing device 100 is the server that performs the electrocardiogram reading, the computing device 100 may obtain electrocardiogram data of a person who wants to read an electrocardiogram by communicating with equipment in a medical environment that measures the electrocardiogram data of the patient or a wearable device capable of measuring the electrocardiogram data. When the computing device 100 is the equipment in the medical environment that measures the electrocardiogram data of the patient or the wearable device capable of measuring the electrocardiogram data, the computing device 100 may measure an electrocardiogram signal to generate the electrocardiogram data.

The computing device 100 may input the electrocardiogram data to the pre-learned deep learning model to predict the onset or progression of myocardial infarction of a subject whose electrocardiogram data was measured (S220). The computing device 100 may input the electrocardiogram data to the pre-trained deep learning model to generate an output value indicating the degree of stenosis of the coronary artery. The computing device 100 may analyze the degree of stenosis of the coronary artery output through the deep learning model based on clinical evidence to derive the onset or progression of myocardial infarction. The computing device 100 may generate a report including the derived information on the onset or progression of myocardial infarction. In this case, when the computing device 100 is the server that performs the electrocardiogram reading, the computing device 100 may share the report through communication with a client that may check the electrocardiogram reading result. When the computing device 100 has an input/output unit that provides a user interface, the computing device 100 may output the report through the input/output unit.

Various embodiments of the present disclosure described above may be combined with additional embodiments and can be modified within a range that may be understood by those skilled in the art in light of the detailed description set forth above. The embodiments of the present disclosure are illustrative in all respects and should be understood as non-limiting. For example, each component described as a single type may be implemented in a distributed manner, and similarly, components described as distributed may be implemented in a combined form. Accordingly, all changes or modifications derived from the meaning, scope, and equivalent concept of the claims of the present disclosure should be construed as being included in the scope of the present disclosure.

## Claims

1. A method of diagnosing myocardial infarction using an electrocardiogram, performed by a computing device including at least one processor, comprising:
generating training data based on electrocardiogram data of a patient who undergoes coronary angiography; and
training a deep learning model that predicts an onset or progression of myocardial infarction in the patient using the generated training data.

2. The method of claim 1, wherein the generating of the training data based on the electrocardiogram data of the patient who undergoes the coronary angiography includes labeling a degree of stenosis of a coronary artery in the electrocardiogram data of the patient who undergoes the coronary angiography to generate the training data.

3. The method of claim 2, wherein the labeling is performed differently according to the type of coronary artery.

4. The method of claim 3, wherein the training of the deep learning model that predicts the onset or progression of myocardial infarction in the patient using the generated training data includes:
classifying the training data based on at least one of a first criterion indicating demographics, a second criterion indicating a factor affecting the onset or progression of myocardial infarction, a third criterion indicating a type of disease and subject of onset, or a fourth criterion indicating a type of electrocardiogram to generate subgroups; and
training the deep learning model to predict the onset or progression of myocardial infarction for each of the generated subgroups.

5. The method of claim 3, wherein the training of the deep learning model that predicts the onset or progression of myocardial infarction in the patient using the generated training data includes:
selecting a group whose performance is less than or equal to a reference value based on a validation result derived from the training process of the generated subgroups; and
performing additional training on the deep learning model on which the training is performed based on the selected group.

6. The method of claim 5, wherein the additional training is performed based on few shot learning.

7. The method of claim 1, wherein the second criterion includes at least one of a risk factor for disease onset, a medical history, whether one is an early onset patient or not, or a type of chest pain.

8. The method of claim 1, wherein the third criterion includes at least one of a type of myocardial infarction, a type of acute coronary syndrome, or a type of coronary artery.

9. A method of diagnosing myocardial infarction using an electrocardiogram, performed by a computing device including at least one processor, comprising:
acquiring electrocardiogram data; and
inputting the electrocardiogram data to a pre-trained deep learning model to predict an onset or progression of myocardial infarction of a subject whose electrocardiogram data is measured,
wherein the deep learning model is pre-trained to predict the onset or progression of myocardial infarction of a patient using training data generated based on the electrocardiogram data of the patient who undergoes coronary angiography.

10. A computer program that is stored in a computer-readable storage medium and performs operations for diagnosing myocardial infarction using an electrocardiogram when executed on one or more processors, wherein the operations include:
generating training data based on electrocardiogram data of a patient who undergoes coronary angiography; and
training a deep learning model that predicts an onset or progression of myocardial infarction in the patient using the generated training data.

11. A computing device for diagnosing myocardial infarction using an electrocardiogram, comprising:
a processor including at least one core;
a memory including program codes executable in the processor; and
a network unit configured to acquire electrocardiogram data,
wherein the processor generates training data based on electrocardiogram data of a patient who undergoes coronary angiography, and
trains the deep learning model that predicts the onset or progression of myocardial infarction in the patient using the generated training data.
